# EUROPÄISCHE PATENTSCHRIFT

(11) **EP 3 906 903 B1**
(45) Veröffentlichungstag und Bekanntmachung des Hinweises auf die Patenterteilung: **02.10.2024**
(21) Anmeldenummer: 21170178.4
(22) Anmeldetag: 23.04.2021
(51) Int. Cl.: A61F 9/008

(54) **VERFAHREN ZUM BEREITSTELLEN VON STEUERDATEN FÜR EINEN AUGENCHIRURGISCHEN LASER, STEUEREINRICHTUNG, BEHANDLUNGSVORRICHTUNG, COMPUTERPROGRAMM, COMPUTERLESBARES MEDIUM**
METHOD FOR PROVIDING CONTROL DATA FOR AN OPHTHALMIC LASER, CONTROL DEVICE, TREATMENT DEVICE, COMPUTER PROGRAM, COMPUTER READABLE MEDIUM
PROCÉDÉ DE FOURNITURE DES DONNÉES DE COMMANDE POUR UN LASER DE CHIRURGIE OCULAIRE, DISPOSITIF DE COMMANDE, DISPOSITIF DE TRAITEMENT, PROGRAMME INFORMATIQUE, SUPPORT LISIBLE PAR ORDINATEUR

(30) Priorität: 06.05.2020 DE 102020112280
(43) Veröffentlichungstag der Anmeldung: 10.11.2021
(73) Patentinhaber: Schwind Eye-Tech-Solutions GmbH, 63801 Kleinostheim (DE)
(72) Erfinder: ARBA MOSQUERA, Samuel, 63739 Aschaffenburg (DE)
(74) Vertreter: Hofstetter, Schurack & Partner

(56) Entgegenhaltungen:
- WO-A2-94/06356
- DE-A1- 102007 019 813
- DE-A1- 102016 208 012
- DAAMGARD: "Intrastromal Lenticule Rotation for Treatment of Astigmatism Up to 10.00 Diopters Ex Vivo in Human Corneas", 1 July 2019 (2019-07-01), XP055843823, Retrieved from the Internet <URL:http://www.ncbi.nlm.nih.gov/pubmed/31298725> [retrieved on 20210922]
- STODULKA PAVEL ET AL: "Intrastromal Lenticule Rotation for the Treatment of High Astigmatism", JOURNAL OF REFRACTIVE SURGERY, vol. 36, no. 6, 1 June 2020 (2020-06-01), US, pages 415 - 418, XP055843820, ISSN: 1081-597X, Retrieved from the Internet <URL:http://dx.doi.org/10.3928/1081597X-20200506-02> DOI: 10.3928/1081597X-20200506-02

## Beschreibung

Die vorliegende Erfindung betrifft ein Verfahren zum Bereitstellen von Steuerdaten für einen augenchirurgischen Laser. Dabei wird der augenchirurgische Laser für die Abtrennung eines Volumenkörpers aus einer menschlichen oder tierischen Kornea gesteuert. Die Erfindung betrifft außerdem ein Verfahren zum Steuern einer Behandlungsvorrichtung, eine Steuereinrichtung, eine Behandlungsvorrichtung, ein Computerprogramm und ein computerlesbares Medium.

Vorrichtungen und Verfahren zur Steuerung von photoablativen ophthalmologischen Lasern sind im Stand der Technik bekannt. Als Alternative zur Laser-in-situ-Keratomileusis (LASIK) kann eine Kurzschnitt-Lentikelextraktion durchgeführt werden ("small-incision lenticule extraction", SMILE), also eine Methode der refraktären Chirurgie, bei der ein Lentikel der Kornea durch einen peripheren Schnitt entfernt wird. Dabei können zum Beispiel ein gepulster Laser und eine Strahlfokussierungseinrichtung so ausgebildet sein, dass die Laserstrahlpulse in einem innerhalb des organischen Materials gelegenen Fokus eine Photodisruption bewirken. Bei einer symmetrischen Pathologie, also bei einer Kornea, die an einer Stelle zu dick ist und dadurch eine Fehlsichtigkeit bewirkt, kann dadurch ein Stück der Hornhaut entfernt werden, um die Fehlsichtigkeit zu korrigieren. Ist die Hornhaut an einer Stelle zu dünn, so kann ein Stück Hornhaut an anderer Stelle extrahiert und in den zu dünnen Bereich transplantiert werden.

Femtosekunden-Systeme werden in der Kornea-Chirurgie verwendet, um zum Beispiel einen Teil einer Kornea zu transplantieren ("deep anterior lamellar keratoplasty", "DALK"), und/oder eine perforierende Keratoplastik (PKP).

Eine Extraktion eines Lentikel ist jedoch invasiv und kann die Kornea ausdünnen und schwächen. Auch eine Transplantation eines Lentikels ist invasiv und kann die Kornea schwächen und verletzen. Aus der Veröffentlichung von Daamgard et al., In: "J. Refract Surg. 2019; 35(7): 451-458" ist ein Verfahren zum Bereitstellen von Steuerdaten für einen augenchirurgischen Laser beschrieben.

Eine der Erfindung zugrundeliegende Aufgabe ist das Reduzieren einer Schwächung der Kornea beim Korrigieren einer Hornhaut-Pathologie, insbesondere einer symmetrischen Hornhaut-Pathologie.

Die gestellte Aufgabe wird durch die erfindungsgemäßen Verfahren mit den Merkmalen der Ansprüche 1 oder 10 und die erfindungsgemäßen Vorrichtungen gemäß den nebengeordneten Ansprüchen 11, 12, 15 oder 16 gelöst. Vorteilhafte Weiterbildungen sind durch die Unteransprüche gegeben.

Die Erfindung basiert auf der Idee, an einem Korrekturbereich einer Kornea des Auges mit einer Ist-Geometrie, wobei die Kornea im Korrekturbereich einen zu dicken und/oder einen zu dünnen Anteil aufweist, eine Form und eine Position eines Lentikels innerhalb des Korrekturbereichs zu ermitteln, der durch seine Form bei einer Drehung um einen vorgegebenen Drehwinkel die Dicke des zu dicken Anteils reduziert und/oder den zu dünnen Anteil verstärkt. Vorzugsweise kann die Korrektur ohne Extraktion durchgeführt werden, jedoch kann in Abhängigkeit von der Pathologie ein Teil des Lentikels extrahiert und nur der Rest gedreht werden.

Vorzugsweise kann auch der Drehwinkel ermittelt werden. Der Drehwinkel kann vorzugsweise anhand der Hornhaut-Pathologie vorgegeben werden.

Mit anderen Worten erfolgt keine vollständige Extraktion eines Lentikels, idealerweise keine Extraktion, und auch keine Transplantation. Vielmehr wird ein Lentikel wird so geformt und gedreht, dass der zu dicke und/oder der zu dünne Anteil der Kornea durch die Drehung zumindest teilweise ausgeglichen werden. Hierdurch kann die Fehlsichtigkeit korrigiert werden. Die durch das erfindungsgemäße Verfahren bereitgestellten Steuerdaten können eine Behandlungsvorrichtung dahingehend steuern, dass der Eingriff weniger invasiv ist und damit die Kornea weniger verdünnt und/oder weniger geschwächt wird. Mit anderen Worten ermöglichen die erfindungsgemäßen Verfahren und die erfindungsgemäßen Vorrichtungen ein für die Kornea sehr viel schonendere und damit weniger belastende Behandlung der Kornea.

Ein erster Aspekt betrifft ein Verfahren zum Bereitstellen von Steuerdaten für einen augenchirurgischen Laser, wobei das Verfahren zumindest die im Folgenden beschriebenen und durch wenigstens eine Steuereinrichtung durchgeführten Schritte aufweist. Unter einer Steuereinrichtung wird dabei ein Gerät, eine Gerätekomponente oder eine Gerätegruppe verstanden, das/die zum Empfangen und Auswerten von Signalen eingerichtet ist, sowie zum Bereitstellen, zum Beispiel Erzeugen, von Steuerdaten. Die Steuereinrichtung kann zum Beispiel als Steuerchip, Computerprogramm, Computerprogrammprodukt oder Steuergerät ausgestaltet sein.

Durch die Steuereinrichtung erfolgt ein Feststellen von Fehlsichtigkeitsdaten, die einen Grad einer Fehlsichtigkeit eines menschlichen oder tierischen Auges beschreiben. Die Fehlsichtigkeitsdaten können zum Beispiel eine Ametropie beschreiben, also ein Abweichen der Brechkraft eines Augapfels vom Idealwert, zum Beispiel eine Kurzsichtigkeit, Weitsichtigkeit oder Stabsichtigkeit. Die Fehlsichtigkeitsdaten können zum Beispiel einen Wert einer Brechkraft beschreiben, also eine Angabe in Dioptrien, oder zum Beispiel eine Abweichung einer Hornhautkrümmung von einem Normalwert. Das Feststellen der Fehlsichtigkeitsdaten kann zum Beispiel durch Abrufen der Fehlsichtigkeitsdaten von einem Datenspeicher oder Datenserver erfolgen, oder die Fehlsichtigkeitsdaten können zum Beispiel aus einem Messgerät, das Abmessungen der Hornhaut vornimmt und/oder die Ametropie feststellt, empfangen werden.

Durch die Steuereinrichtung erfolgt ein Feststellen einer Ist-Geometrie eines Korrekturbereichs einer Kornea des Auges, die dreidimensionale Abmessungen des Korrekturbereichs der Kornea beschreibt. Die Ist-Geometrie kann zum Beispiel durch Scannen und Vermessen der Kornea festgestellt werden, oder durch Bereitstellen solcher Messdaten. Unter der Ist-Geometrie wird also eine tatsächliche, aktuelle Geometrie der Kornea verstanden. Der Korrekturbereich ist dabei derjenige Bereich der Kornea, der zum Beheben oder Reduzieren der Fehlsichtigkeit korrigiert werden soll.

Anhand der festgestellten Fehlsichtigkeitsdaten und anhand der festgestellten Ist-Geometrie erfolgt durch die Steuereinrichtung ein Ermitteln einer Soll-Geometrie des Korrekturbereichs, wobei die Soll-Geometrie mindestens einen Korrekturanteil des Korrekturbereichs beschreibt, also mindestens einen Anteil des Korrekturbereichs, der eine zu dicke oder zu dünne Kornea hat. Der Korrekturanteil kann bereits durch die Ist-Geometrie beschrieben sein, oder zum Beispiel anhand der Ist-Geometrie festgestellt werden. Die Soll-Geometrie beschreibt also die gewünschte Geometrie der zu korrigierenden Stellen.

Die Soll-Geometrie kann, mit anderen Worten, einen Reduktionsanteil des Korrekturbereichs mit einer gegenüber der Ist-Geometrie verminderten oder reduzierten Kornea-Dicke beschreiben, mit anderen Worten einen Anteil der Kornea, der gemäß der Ist-Geometrie zu dick ist und gemäß der Soll-Geometrie durch Reduzieren der Dicke korrigiert ist. Die Soll-Geometrie beschreibt alternativ oder zusätzlich einen Verdickungsanteil mit einer gegenüber der Ist-Geometrie erhöhten Kornea-Dicke. Der Verdickungsanteil ist derjenige Teil der Kornea, der gemäß der Ist-Geometrie zu dünn ist und zum Beheben oder Reduzieren der Fehlsichtigkeit verdickt werden soll. Die ermittelte Soll-Geometrie erfüllt dabei ein vorgegebenes Fehlsichtigkeitsreduktions-Kriterium, welches eine Eignung zur Reduktion des Grads der Fehlsichtigkeit vorgibt. Hierzu kann das vorgegebene Fehlsichtigkeitsreduktions-Kriterium zum Beispiel eine Reduktion der Fehlsichtigkeit um 0,5 Dioptrien beschreiben, oder zum Beispiel eine Reduktion der Fehlsichtigkeit um 50% oder 100%, oder eine möglichst hohe Reduktion der Fehlsichtigkeit.

Anhand der festgestellten Ist-Geometrie ermittelt die Steuereinrichtung Grenzflächen eines Lentikels innerhalb des Korrekturbereichs, der sich zumindest teilweise über den Korrekturbereich erstreckt, und wobei ein Anteil des Lentikels derart ausgestaltet ist, dass er bei einer Drehung in dem Verdickungsanteil um eine vorgegebene, durch die Kornea verlaufende Drehachse und um einen vorgegebenen Drehwinkel den Korrekturbereich von der festgestellten Ist-Geometrie in die ermittelte Soll-Geometrie überführt. Unter einem Lentikel wird dabei ein Volumenkörper der Kornea verstanden, also zum Beispiel eine disk-förmige Scheibe. Der Lentikel oder Volumenkörper kann optional linsenförmig sein, alternativ zum Beispiel bohnenförmig. Der Lentikel kann symmetrisch oder asymmetrisch geformt sein. Die Drehachse ist dabei eine Achse, die durch einen vorbestimmten Drehpunkt des Lentikels führt, wobei die Drehachse zum Beispiel zwischen dem hinteren Pol des Augapfels und dem vorderen Pol des Augapfels verlaufen kann, also zum Beispiel auf der optischen Achse liegen kann.

Die Steuereinrichtung stellt Steuerdaten bereit, kann diese also zum Beispiel erzeugen, wobei die bereitgestellten Steuerdaten die ermittelten Grenzflächen zum Formen des Lentikels beschreiben. Die Steuerdaten können zum Beispiel Koordinaten der Grenzflächen beschreiben, und/oder Vektoren, entlang denen der Laser die Grenzflächen schneiden soll. Die Grenzflächen können zum Beispiel einen bikonvexen, bikonkaven, plankonvexen oder plankonkaven Volumenkörper ausbilden.

Es ergeben sich die oben genannten Vorteile. Die Form des Lentikels trägt nach der Drehung um den vorgegebenen Drehwinkel zur Korrektur der Fehlsichtigkeit des Auges bei. Durch die bereitgestellten Steuerdaten kann ein Augenlaser also derart gesteuert werden, dass der Lentikel entsprechend geschnitten und gedreht wird. Durch die Drehung um den vorgegebenen Drehwinkel werden Verdickungsbereich und/oder Reduktionsbereich ausgeglichen. Eine vollständige Extraktion des Lentikels ist nicht nötig, wodurch die Kornea deutlich weniger geschwächt wird. Der Lentikel kann zum Beispiel durch Lasern der ermittelten Grenzflächen abgetrennt, gedreht und wieder angedrückt werden. Falls überhaupt ein Anteil des Lentikels extrahiert werden muss, ist dieser Anteil sehr klein, weswegen eine Teil-Extraktion weniger invasiv ist als eine Extraktion des gesamten Lentikels und eine Lentikel-Transplantation. Idealerweise kann eine Extraktion sogar ganz vermieden werden.

Die bereitgestellten Steuerdaten können optional die vorgegebene Drehachse und eine Rotation des Lentikels um den vorgegebenen Drehwinkel beschreiben. Dadurch kann auch die Drehung des Lentikels durch die Behandlungsvorrichtung durchgeführt werden, falls diese zum Beispiel ein entsprechendes Instrument umfasst, zum Beispiel eine Pinzette oder Mikro-Pinzette. Dadurch wird eine besonders präzise Feinjustierung des gedrehten Lentikels ermöglicht.

Die Steuereinrichtung kann vorzugsweise ein Festlegen von mindestens einem Inzisionsverlauf durchführen, der sich jeweils von einer vorgegebenen Inzisionsstelle an der Außenseite der Kornea zu einer der ermittelten Grenzflächen hin erstreckt. Mit anderen Worten ist der Inzisionsverlauf derjenige Verlauf oder diejenige Linie, der/die zum Beispiel als Zugang für ein Instrument zum Drehen des Lentikels benutzt werden kann, und/oder ein Zugang, über den zum Beispiel ein Teil des Lentikels extrahiert werden kann. Die bereitgestellten Steuerdaten können dabei dann den mindestens einen festgelegten Inzisionsverlauf beschreiben. Auch hierdurch wird eine besonders präzise Feinjustierung des gedrehten Lentikels ermöglicht.

Vorzugsweise kann die Steuereinrichtung den Drehwinkel vorgeben und/oder die Drehachse. Dies kann jeweils in Abhängigkeit von a) einem Ergebnis eines Vergleichs der festgestellten Ist-Geometrie mit der ermittelten Soll-Geometrie erfolgen, und/oder b) in Abhängigkeit von den festgestellten Fehlsichtigkeitsdaten. Mit anderen Worten kann die Steuereinrichtung zum Beispiel berechnen, zum Beispiel anhand der Form des Lentikels und anhand des Unterschieds zwischen der Ist-Geometrie und der Soll-Geometrie, wie weit der Lentikel gedreht werden muss, um die Fehler im Reduktionsanteil und/oder im Verdickungsanteil auszugleichen. Hierdurch kann die Korrektur der Fehlsichtigkeit sehr viel präziser erfolgen.

Vorzugsweise kann die Steuereinrichtung eine Soll-Endposition eines vorgegebenen Lentikelbereichs des Lentikels feststellen, mit anderen Worten diejenige Endposition, in der ein Anteil des Lentikels nach dem Drehen gelagert sein soll. Die bereitgestellten Steuerdaten können dann ein Markieren der festgestellten Soll-Endposition außerhalb des Lentikels beschreiben; und/oder die bereitgestellten Steuerdaten können ein Markieren des vorgegebenen Lentikelbereichs beschreiben. Mit anderen Worten können die Steuerdaten zum Beispiel beschreiben, dass der Lentikel an einer bestimmten Stelle markiert wird, zum Beispiel durch Einlasern eines Fadenkreuzes; und/oder die bereitgestellten Steuerdaten können das Anbringen einer entsprechenden Markierung außerhalb des Lentikels, also im Restkörper der Kornea, beschreiben. Sind zum Beispiel sowohl der Lentikelbereich markiert, als auch eine Stelle außerhalb des Lentikels, so kann durch die bereitgestellten Steuerdaten das Markieren derart erfolgen, dass ein Chirurg weiß, dass der Lentikel so weitergedreht werden muss, dass die Markierung auf dem Lentikelbereich neben der Markierung am Korneakörper anliegt. Dies unterstützt nicht nur ein Drehen des Lentikels, falls dies durch einen Chirurgen durchgeführt wird, sondern ein Abgleich solcher Markierungen kann auch durch die Behandlungsvorrichtung herangezogen werden, um die optimale Position des gedrehten Lentikels zu verifizieren.

In einer Weiterbildung können die bereitgestellten Steuerdaten ein Markieren des Lentikelbereichs an dessen Koordinaten durch einen Augenlaser beschreiben. Der Augenlaser kann zum Beispiel ein Fadenkreuz als Markierung anbringen. Eine solche Markierung ist nicht nur minimalinvasiv, sondern auch gut überprüfbar.

Gemäß einer weiteren Ausführungsform des erfindungsgemäßen Verfahrens können die Grenzflächen den Lentikel von der Kornea vollständig abtrennen. Mit anderen Worten können die Steuerdaten Grenzflächen beschreiben, die einen durchgängig abgetrennten Lentikel formen. Ein solcher vollständig abgetrennter Lentikel ist einfach zu drehen und kann leicht verschoben werden.

In manchen Fällen kann es jedoch wünschenswert sein, dass der Lentikel weniger einfach verschiebbar ist, zum Beispiel in Fällen, in denen der Lentikel in seiner Endposition und durch seine Form bedingt zum Beispiel weniger rutschfest im Korneakörper gelagert ist. Ein anderer Fall ist zum Beispiel, falls ein Teil des Lentikels extrahiert wird und der verbleibende Anteil des Lentikels gedreht wird. In solchen Fällen ist es besonders vorteilhaft, falls die ermittelten Grenzflächen den Lentikel nur außerhalb eines Drehbereichs des Lentikels, durch den die vorgegebene Drehachse verläuft, aus dem Korrekturbereich der Kornea abtrennen, sodass der Lentikel im Drehbereich mit dem an der Lederhaut verbundenen Körper der Kornea verbunden bleibt. Der an der Lederhaut verbundene Körper der Kornea ist dabei der Restkörper der Kornea, der den Lentikel umgibt. Der Drehbereich kann zum Beispiel zylinderförmig sein. Dadurch, dass bei dieser Ausführungsform des erfindungsgemäßen Verfahrens der Lentikel über den Drehbereich mit dem Restkörper der Kornea verbunden bleibt, kann das Drehen zum Beispiel nur mit einer Pinzette statt mehrerer Pinzetten durchgeführt werden. Dadurch ist das Verfahren weniger invasiv, die Kornea wird also weniger belastet. Eine Wahrscheinlichkeit, dass der Lentikel in seiner Endposition verrutscht, wird deutlich reduziert oder sogar vermieden. Ein weiterer Vorteil ist, dass der Lentikel bei der Drehung die gleiche Zentrierung aufweist.

Gemäß einem weiteren, bevorzugten Ausführungsbeispiel des erfindungsgemäßen Verfahrens kann die Steuereinrichtung ein Ermitteln von Grenzflächen eines weiteren, zu extrahierenden Lentikels durchführen, vorzugsweise anhand eines Ergebnisses eines Vergleichs der ermittelten Soll-Geometrie und der festgestellten Ist-Geometrie, wobei sich der zu extrahierende Lentikel vorzugsweise nur in einem Reduktionsanteil befindet, und wobei die bereitgestellten Steuerdaten die Grenzflächen des weiteren Lentikels beschreiben. Alternativ kann der zu extrahierende Lentikel auch als zu extrahierender Lentikelanteil des zuvor ermittelten Lentikels definiert sein. Mit anderen Worten kann von dem ermittelten, zu drehenden Lentikel ein Anteil festgestellt werden, der extrahiert werden soll, bevor, während oder nach der verbleibende Anteil gedreht wird. Im Reduktionsanteil kann also ein Stück des Lentikels entnommen werden, sodass der entnommene Anteil nach dem Drehen den Korrekturbereich an anderer Stelle nicht verdickt. Hierdurch kann eine zu dicke Stelle ausgeglichen werden, ohne dass an anderer Stelle die Hornhautdicke zu sehr verstärkt wird. Diese Ausführungsform ermöglicht eine noch flexiblere Gestaltung bei der Fehlsichtigkeitskorrektur. Außerdem wird so mehr Platz für die Rotation des anderen Lentikels oder des verbleibenden Lentikelbereichs geschaffen.

In weiteren vorteilhaften Ausgestaltungen des erfindungsgemäßen Verfahrens ist die Steuereinrichtung derart ausgebildet, dass der Laser Laserpulse in einem Wellenlängenbereich zwischen 300 Nanometer (nm) und 1400 nm, vorzugsweise zwischen 700 nm und 1200 nm, bei einer jeweiligen Pulsdauer zwischen 1 Femtosekunde (fs) und 1 Nanosekunde (ns), vorzugsweise zwischen 10 fs und 10 Pikosekunden (ps), und einer Wiederholungsfrequenz größer 10 Kilohertz (KHz), vorzugsweise zwischen 100 KHz und 100 Megahertz (MHz), abgibt. Derartige Laser werden bereits für photodisruptive Verfahren in der Augenchirurgie verwendet. Ein solcher Femtosekundenlaser ist zur Herstellung von Volumenkörpern innerhalb der Kornea besonders gut geeignet. Die Verwendung von photodisruptiven Lasern bei dem erfindungsgemäßen Verfahren weist zudem den Vorteil auf, dass die Bestrahlung der Kornea nicht in einem Wellenlängenbereich unter 300 nm erfolgen muss. Dieser Bereich wird in der Lasertechnik unter dem Begriff "tiefes Ultraviolett" subsumiert. Dadurch wird vorteilhafterweise vermieden, dass durch diese sehr kurzwelligen und energiereichen Strahlen eine unbeabsichtigte Schädigung der Kornea erfolgt. Photodisruptive Laser der hier verwendeten Art bringen üblicherweise gepulste Laserstrahlung mit einer Pulsdauer zwischen 1 fs und 1 ns in das Korneagewebe ein. Dadurch kann die für den optischen Durchbruch notwendige Leistungsdichte des jeweiligen Laserpulses räumlich eng begrenzt werden, so dass eine hohe Schnittgenauigkeit bei der Erzeugung der Grenzflächen ermöglicht wird. Als Wellenlängenbereich kann insbesondere auch der Bereich zwischen 700 nm und 780 nm gewählt werden.

Ein weiterer Aspekt der Erfindung betrifft ein Verfahren zum Steuern einer Behandlungsvorrichtung, wobei das Verfahren die Verfahrensschritte einer der Ausführungsformen des Verfahrens des ersten Aspekts der Erfindung umfasst, sowie ein Übertragen der bereitgestellten Steuerdaten an einen augenchirurgischen Laser der Behandlungsvorrichtung. Es werden die bereits oben genannten Vorteile erreicht.

Ein dritter Aspekt der vorliegenden Erfindung betrifft eine Steuereinrichtung, die dazu eingerichtet ist, eines der oben beschriebenen Verfahren durchzuführen. Es ergeben sich die oben aufgeführten Vorteile. Die Steuereinrichtung kann zum Beispiel als Steuerchip, Steuergerät oder Anwenderprogramm ("App") ausgestaltet sein. Die Steuereinrichtung kann vorzugsweise eine Prozessoreinrichtung aufweisen und/oder einen Datenspeicher. Unter einer Prozessoreinrichtung wird ein Gerät oder eine Gerätekomponente zur elektronischen Datenverarbeitung verstanden. Die Prozessoreinrichtung kann zum Beispiel mindestens einen Mikrocontroller und/oder mindestens einen Mikroprozessor aufweisen. Auf dem optionalen Datenspeicher kann vorzugsweise ein Programmcode zum Durchführen des erfindungsgemäßen Verfahrens abgelegt sein. Der Programmcode kann dann dazu ausgelegt sein, bei Ausführung durch die Prozessoreinrichtung die Steuereinrichtung dazu zu veranlassen, eine der oben beschriebenen Ausführungsformen des erfindungsgemäßen Verfahrens durchzuführen.

Ein vierter Aspekt der vorliegenden Erfindung betrifft eine Behandlungsvorrichtung mit mindestens einem augenchirurgischen Laser für die Abtrennung eines vordefinierten Hornhautvolumens mit vordefinierten Grenzflächen eines menschlichen oder tierischen Auges mittels Photodisruption und mindestens einer Steuereinrichtung für den oder die Laser, die ausgebildet ist, die Schritte des Verfahrens gemäß dem ersten Aspekt der Erfindung auszuführen, und/oder die Schritte des Verfahrens gemäß dem zweiten Aspekt der Erfindung. Die erfindungsgemäße Behandlungsvorrichtung ermöglicht es, dass die bei der Verwendung üblicher ablativer Behandlungsvorrichtungen auftretenden Nachteile zuverlässig reduziert oder sogar vermieden werden.

In einer weiteren vorteilhaften Ausgestaltung der erfindungsgemäßen Behandlungsvorrichtung kann der Laser dazu geeignet sein, Laserpulse in einem Wellenlängenbereich zwischen 300 nm und 1400 nm, vorzugsweise zwischen 700 nm und 1200 nm, bei einer jeweiligen Pulsdauer zwischen 1 fs und 1 ns, vorzugsweise zwischen 10 fs und 10 ps, und einer Wiederholungsfrequenz größer 10 Kilohertz (KHz), vorzugsweise zwischen 100 KHz und 100 Megahertz (MHz), abzugeben. Es ergeben sich die bereits oben genannten Vorteile.

In weiteren vorteilhaften Ausgestaltungen der erfindungsgemäßen Behandlungsvorrichtung kann die Steuereinrichtung mindestens eine Speichereinrichtung zur zumindest temporären Speicherung von mindestens einem Steuerdatensatz aufweisen, wobei der oder die Steuerdatensätze Steuerdaten zur Positionierung und/oder zur Fokussierung einzelner Laserpulse in der Hornhaut/Kornea umfassen; und kann mindestens eine Strahleinrichtung zur Strahlführung und/oder Strahlformung und/oder Strahlablenkung und/oder Strahlfokussierung eines Laserstrahls des Lasers aufweisen. Die genannten Steuerdatensätze werden dabei üblicherweise anhand einer gemessenen Topografie und/oder Pachymetrie und/oder Morphologie der zu behandelnden Kornea und der Art der zu korrigierenden Fehlsichtigkeit, erzeugt.

Weitere Merkmale und deren Vorteile sind den Beschreibungen des ersten Erfindungsaspekts zu entnehmen, wobei vorteilhafte Ausgestaltungen jedes Erfindungsaspekts als vorteilhafte Ausgestaltungen des jeweils anderen Erfindungsaspekts anzusehen sind.

Ein fünfter Aspekt der Erfindung betrifft ein Computerprogramm, umfassend Befehle, die bewirken, dass die Behandlungsvorrichtung gemäß dem vierten Erfindungsaspekt die Verfahrensschritte gemäß dem ersten Erfindungsaspekt und/oder die Verfahrensschritte gemäß dem zweiten Erfindungsaspekt ausführt.

Ein sechster Aspekt der Erfindung betrifft ein computerlesbares Medium, auf dem das Computerprogramm gemäß dem fünften Erfindungsaspekt gespeichert ist. Weitere Merkmale und deren Vorteile sind den Beschreibungen des ersten bis vierten Erfindungsaspekts zu entnehmen, wobei vorteilhafte Ausgestaltungen jedes Erfindungsaspekts als vorteilhafte Ausgestaltungen des jeweils anderen Erfindungsaspekts anzusehen sind.

Weitere Merkmale der Erfindung ergeben sich aus den Ansprüchen, den Figuren und der Figurenbeschreibung. Die vorstehend in der Beschreibung genannten Merkmale und Merkmalskombinationen, sowie die nachfolgend in der Figurenbeschreibung genannten und/oder in den Figuren alleine gezeigten Merkmale und Merkmalskombinationen sind nicht nur in der jeweils angegebenen Kombination, sondern auch in anderen Kombinationen verwendbar, ohne den Rahmen der Erfindung zu verlassen. Es sind somit auch Ausführungen von der Erfindung als umfasst und offenbart anzusehen, die in den Figuren nicht explizit gezeigt und erläutert sind, jedoch durch separierte Merkmalskombinationen aus den erläuterten Ausführungen hervorgehen und erzeugbar sind. Es sind auch Ausführungen und Merkmalskombinationen als offenbart anzusehen, die somit nicht alle Merkmale eines ursprünglich formulierten unabhängigen Anspruchs aufweisen. Es sind darüber hinaus Ausführungen und Merkmalskombinationen, insbesondere durch die oben dargelegten Ausführungen, als offenbart anzusehen, die über die in den Rückbezügen der Ansprüche dargelegten Merkmalskombinationen hinausgehen oder von diesen abweichen. Dabei zeigt
- Fig. 1: eine schematische Darstellung einer erfindungsgemäßen Behandlungsvorrichtung;
- Fig. 2: eine Prinzipdarstellung der Erzeugung eines zu drehenden Volumenkörpers;
- Fig. 3: eine schematische dreidimensionale Darstellung einer Kornea mit einem Keratokonus und einem zu drehenden Volumenkörper; und
- Fig. 4: eine schematische dreidimensionale Darstellung einer verkrümmten Kornea und einem zu drehenden Volumenkörper.

In den Figuren sind gleiche oder funktionsgleiche Elemente mit den gleichen Bezugszeichen versehen.

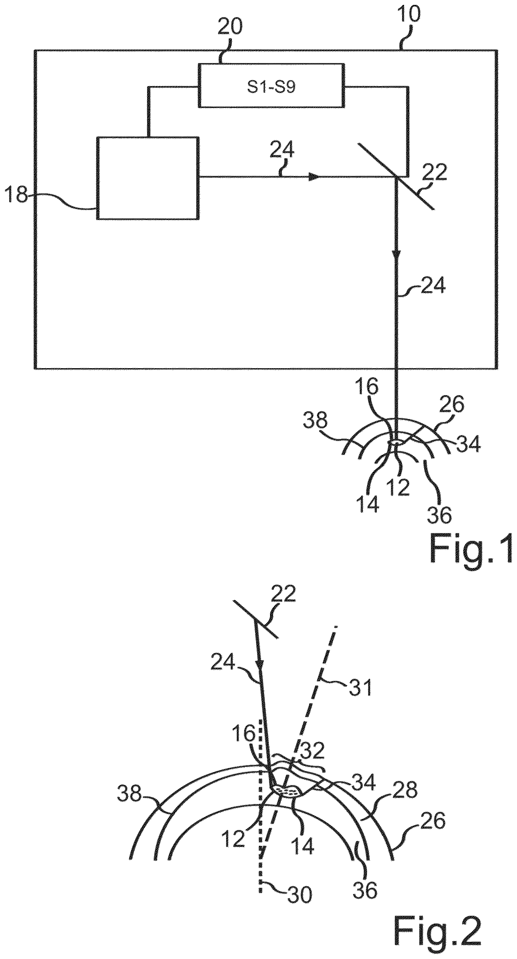

Die Figur 1 zeigt eine schematische Darstellung einer Behandlungsvorrichtung 10 mit einem augenchirurgischen Laser 18 für die Abtrennung eines vordefinierten Hornhautvolumens/Korneavolumens, also eines Lentikels 12 einer Kornea 17 eines menschlichen oder tierischen Auges, der auch als Volumenkörper bezeichnet werden kann, mit vordefinierten Grenzflächen 14, 16 mittels Photodisruption. Man erkennt, dass neben dem Laser 18 eine Steuereinrichtung 20 für den Laser 18 ausgebildet sein kann, sodass dieser gepulste Laserpulse beispielsweise in einem vordefinierten Muster in die Kornea 17 abgibt, wobei die ermittelten Grenzflächen 14, 16 des zu bildenden Lentikels 12 durch zum Beispiel ein vordefiniertes Muster mittels Photodisruption erzeugt werden können. Alternativ kann die Steuereinrichtung 20 eine in Bezug auf die Behandlungsvorrichtung 10 externe Steuereinrichtung 20 sein.

Die ermittelten Grenzflächen 14, 16 bilden in dem dargestellten Ausführungsbeispiel einen Lentikel 12 aus, wobei die Position des Lentikels 12 in diesem Ausführungsbeispiel derart gewählt sein kann, dass dieser zumindest teilweise in einem krankhaft und/oder unnatürlich veränderten Korrekturbereich 32 (siehe Figur 2) liegt, zum Beispiel innerhalb einer Stroma 36 der Kornea 17. Des Weiteren ist aus Figur 1 erkennbar, dass zwischen der Stroma 36 und einem Epithelium 28 die so genannte Bowman-Membran 38 ausgebildet ist. Alternativ zu dem in den Figuren 1 und 2 gezeigten Beispielen kann der Lentikel 12 durch die Grenzflächen 14, 16 nicht vollständig abgetrennt werden. Mit anderen Worten kann der Lentikel 12 nach dem Lasern der Grenzflächen 14, 16 in einem Drehbereich mit dem Rest der Kornea 17 verbunden bleiben.

Des Weiteren zeigen die Figur 1 und die Figur 2, dass der durch den Laser 18 erzeugte Laserstrahl 24 mittels einer Strahleinrichtung 22, nämlich einer Strahlablenkungsvorrichtung, wie zum Beispiel einem Rotationscanner, in Richtung einer Oberfläche 26 der Hornhaut abgelenkt wird. Die Strahlablenkvorrichtung wird ebenfalls durch die Steuereinrichtung 20 gesteuert, um die ermittelten Grenzflächen 14, 16, vorzugsweise auch einen Schnitt 34 entlang von einem vorgegebenen Inzisionsverlauf oder mehrere Schnitte 34 entlang von vorgegebenen Inzisionsverläufen, zu erzeugen.

Bei dem dargestellten Laser 18 kann es sich vorzugsweise um einen photodisruptiven Laser handeln, der ausgebildet ist, Laserpulse in einem Wellenlängenbereich zwischen 300 nm und 1400 nm, vorzugsweise zwischen 700 nm und 1200 nm, bei einer jeweiligen Pulsdauer zwischen 1 fs und 1 ns, vorzugsweise zwischen 10 fs und 10 ps, und einer Wiederholungsfrequenz größer 10 KHz, vorzugsweise zwischen 100 KHz und 100 MHz, abzugeben. Die Steuereinrichtung 20 weist optional zudem eine Speichereinrichtung (nicht dargestellt) zur zumindest temporären Speicherung von mindestens einem Steuerdatensatz auf, wobei der oder die Steuerdatensätze Steuerdaten zur Positionierung und/oder zur Fokussierung einzelner Laserpulse in der Kornea 17 umfassen. Die Positionsdaten und/oder Fokussierungsdaten der einzelnen Laserpulse werden anhand einer zuvor gemessenen Topografie und/oder Pachymetrie und/oder der Morphologie der Hornhaut und dem beispielsweise zu entfernenden, krankhaften und/oder unnatürlich veränderten Korrekturbereich 32 oder der zu erzeugenden optischen Fehlsichtigkeitskorrektur beispielhaft innerhalb der Stroma 36 des Auges erzeugt, vorzugsweise anhand einer festgestellten Ist-Geometrie der Kornea 17 des Korrekturbereichs 32 und anhand einer Analyse, wie der Lentikel 12 zur Korrektur der Fehlsichtigkeit ausgestaltet sein soll.

Die Figur 2 zeigt eine Prinzipdarstellung der Erzeugung des zu drehenden Lentikels 12 gemäß einer Ausführungsform des vorliegenden Verfahrens. Man erkennt, dass mittels des gepulsten Laserstrahls 24, der über die Strahlablenkvorrichtung 22 in Richtung der Kornea 17 beziehungsweise in Richtung der Oberfläche 26 der Kornea 17 gelenkt wird, die ermittelten Grenzflächen 14, 16 erzeugt werden. Die ermittelten Grenzflächen 14, 16 können dabei einen Lentikel 12 ausbilden, der zumindest teilweise im krankhaften und/oder unnatürlich veränderten Korrekturbereich 32 liegt. Des Weiteren erzeugt in dem dargestellten Ausführungsbeispiel der Laser 18 einen weiteren Schnitt 34 entlang einem Inzisionsverlauf, der in einem vordefinierten Winkel und mit einer vordefinierten Geometrie den Lentikel 12 schneidet und bis zu der Oberfläche 26 der Kornea 17 ausgebildet ist. Der durch die Grenzflächen 14, 16 definierte Lentikel 12 kann dann über den Schnitt 34 aus der Kornea 17 gedreht werden. Optional kann ein Teil des Lentikels 12 extrahiert werden.

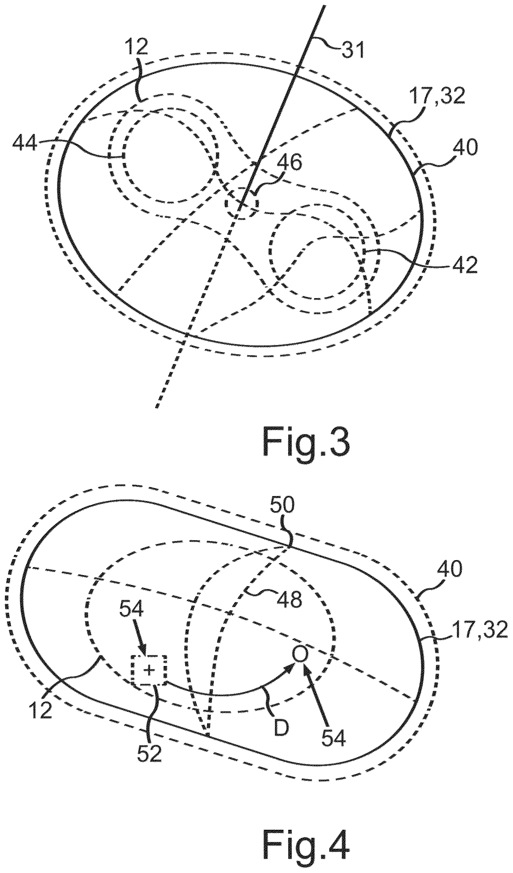

In dem dargestellten Ausführungsbeispiel kann der krankhafte und/oder unnatürlich veränderte Korrekturbereich 32 zum Beispiel innerhalb der Stroma 36 und außerhalb einer optischen Achse 30 eines Auges 40 (Figur 3) ausgebildet sein.

In dem dargestellten Ausführungsbeispiel kann zunächst mittels des Laserstrahls 24 die Grenzfläche 14, das heißt die tiefer im Auge 40 beziehungsweise der Stroma 36 liegende Grenzfläche ausgebildet werden, wobei diese dann der posterioren Grenzfläche 14 entspricht. Dies kann durch ein zumindest teilweise kreis- und/oder spiralförmiges Führen des Laserstrahls 24 erfolgen. Anschließend wird auf vergleichbare Art und Weise die Grenzfläche 16 erzeugt, welche dann der anterioren Grenzfläche 16 entspricht, sodass die Grenzflächen 14, 16 den Lentikel 12 (siehe auch Figur 1) ausbilden. Anschließend wird der Schnitt 34 ebenfalls mit dem Laser 18 erzeugt. Die Reihenfolge der Erzeugung der Grenzflächen 14, 16 und des Schnitts 34 kann jedoch auch geändert werden.

Die Drehachse 31 zum Drehen D des Lentikels 12 kann entsprechend vorgegeben sein. Nach dem Lasern der Grenzflächen 14, 16 kann die Drehung D um einen vorgegebenen Drehwinkel um die Drehachse 31 zum Beispiel durch eine oder mehrere Mikropinzetten, die beispielsweise ein Bauteil der Behandlungsvorrichtung 10 sein kann, oder mittels anderen geeigneten Instrumenten durchgeführt werden. Die Mikropinzetten (in den Figuren nicht gezeigt) können zum Beispiel so gesteuert werden, dass sie durch den Schnitt 34 oder durch die Schnitte 34 geführt werden, um zum Lentikel 12 zu gelangen.

Das Verfahren zum Bereitstellen entsprechender Steuerdaten wird im Zusammenhang mit dem Beispiel der Figur 3 und der Figur 4 näher erläutert. Im Beispiel der Figur 3 kann die Kornea 17 zum Beispiel einen Keratokonus bilden. Ein solcher Keratokonus, also eine lokale Aufsteilung der Kornea 17, kann sich dadurch bilden, dass die Kornea 17 an dieser Stelle so dünn ist, dass sich die Kornea 17 durch den Druck des Massekörpers des Auges 40, also durch den Augeninnendruck, nach außen wölbt. Der Bereich um den Keratokonus kann dann im Beispiel der Fig. 3 als Verdickungsanteil 42 definiert oder vorgegeben werden, also als derjenige Anteil des Korrekturbereichs 32, der zur Korrektur der Fehlsichtigkeit verdickt werden soll, mit anderen Worten dessen Dicke durch die Korrektur erhöht werden soll.

Im Beispiel der Fig. 3 ist ebenfalls ein Reduktionsanteil 44 gezeigt, also ein Anteil des Korrekturbereichs 32, der zu dick ist und zum Reduzieren einer Hornhautverkrümmung im Reduktionsanteil 44 dünner ausgestaltet werden soll, dessen Dicke also reduziert werden soll.

Zum Feststellen von Fehlsichtigkeitsdaten (Verfahrensschritt S1, vergleiche Fig. 1), die zum Beispiel einen Wert in Dioptrien angeben können oder andere, geeignete Daten zum Beschreiben der Fehlsichtigkeit, kann die Steuereinrichtung 20 zum Beispiel die entsprechenden Daten von einem Datenserver empfangen, oder die Fehlsichtigkeitsdaten können als Dateneingabe festgestellt werden. Die Ist-Geometrie des Korrekturbereichs 32, also eine Geometrie des Korrekturbereichs 32 vor einem Eingriff, die die Fehlsichtigkeit verursacht, kann festgestellt werden (S2), indem zum Beispiel der Korrekturbereich 32 mit dem Fachmann aus dem Stand der Technik bekannten Mitteln vermessen wird. Optional können die festgestellten Fehlsichtigkeitsdaten die Ist-Geometrie bereits beschreiben.

Optional kann anhand der festgestellten Fehlsichtigkeitsdaten und/oder der festgestellten Ist-Geometrie ein dreidimensionales, vorzugsweise digitales Modell der Kornea 17 und/oder des Korrekturbereichs 32 bereitgestellt werden. Beispielsweise anhand eines solchen Modells kann die Soll-Geometrie des Korrekturbereichs 32 ermittelt werden (S3). Die Soll-Geometrie beschreibt dabei auch zum Beispiel den Reduktionsanteil 44 und den Verdickungsanteil 42. Die Soll-Geometrie erfüllt ein vorgegebenes Fehlsichtigkeitsreduktions-Kriterium, also die Vorgabe, dass die festgestellte Fehlsichtigkeit durch die Soll-Geometrie reduziert oder sogar ganz behoben wird. Optional kann das Fehlsichtigkeitsreduktions-Kriterium zum Beispiel einen Mindestwert einer solchen Reduktion vorgeben, oder eine Mindestwahrscheinlichkeit, mit der die Fehlsichtigkeit reduziert wird. Das Fehlsichtigkeitsreduktions-Kriterium kann zum Beispiel in der Speichereinrichtung abgelegt sein.

Anhand der festgestellten Ist-Geometrie, vorzugsweise unter Berücksichtigung der ermittelten Soll-Geometrie, kann die Steuereinrichtung 20 nun die Grenzflächen 14, 16 ermitteln (S4). Hierzu kann die Steuereinrichtung 20 zum Beispiel zunächst die Form des Lentikels 12 berechnen. Optional kann die Steuereinrichtung 20 zunächst mehrere, mögliche Positionen und Formen mehrerer Lentikel 12 feststellen und für jeden der möglichen Lentikel 12 einen Grad der Reduktion der Fehlsichtigkeit prädizieren. Diejenige Form und/oder Position eines Lentikels 12, die dann zum Beispiel den höchsten Grad einer Reduktion der Fehlsichtigkeit verspricht, kann dann ausgewählt werden als Vorlage zum Ermitteln der Grenzflächen 14, 16 (S4).

Aufgrund der dreidimensionalen Darstellung der Fig. 3 sind die anteriore Grenzfläche 14 und die posteriore Grenzfläche 16 nicht eingezeichnet. Die Fig. 3 zeigt jedoch einen beispielhaften Umriss eines Lentikels 12, bei dessen Drehung um zum Beispiel 180 Grad um die Drehachse 31 der gemäß der Ist-Geometrie am Reduktionsanteil 44 liegende Lentikelbereich an die Stelle des Verdickungsbereichs 42 übergehen kann und dort die Hornhautdicke erhöht, sodass sich der Keratokonus zurückbilden kann. Die ermittelten Grenzflächen 14, 16 können entweder einen vom Rest der Kornea 17 vollständig abgetrennten Lentikel 12 beschreiben, oder aber, wie im Beispiel der Fig. 3 veranschaulicht, einen Lentikel 12, der in einem Drehbereich 46 nicht von dem Rest der Kornea 17 abgetrennt ist.

Das Vorgeben des Drehwinkels und/oder der Drehachse 31 kann die Steuereinrichtung 20 in Abhängigkeit von einem Ergebnis eines Vergleichs der festgestellten Ist-Geometrie mit der ermittelten Soll-Geometrie durchführen, das heißt anhand der Hornhaut-Pathologie. Alternativ oder zusätzlich kann die Steuereinrichtung 20 den Drehwinkel anhand der festgestellten Fehlsichtigkeitsdaten (S5) vorgeben. Stellt die Steuereinrichtung 20 dann im Verfahrensschritt S6 die Steuerdaten bereit, die zum Beispiel Koordinaten der Grenzflächen 14, 16 beschreiben, können die Steuerdaten idealerweise auch den Drehwinkel und/oder die Drehachse vorgeben. Dabei können die Steuerdaten auch zum Beispiel ein Instrument zum Drehen des Lentikels 12 steuern.

Vorzugsweise können die bereitgestellten Steuerdaten auch festgelegte Inzisionsverläufe beschreiben, die dem Laser den Schnitt 34 oder die Schnitte 34 vorgeben. Zum Festlegen einer oder mehrerer Inzisionsverläufe (S7) kann zum Beispiel berücksichtigt werden, dass diese nicht im Drehbereich 46 liegen (Fig. 3).

Ergibt die Analyse der Ist-Geometrie zum Beispiel, dass es sinnvoll ist, einen Anteil der Kornea 17 zu entfernen, bevor, während oder nachdem der ermittelte Lentikel 12 gedreht wird, können zum Beispiel Grenzflächen 14, 16 eines weiteren zu extrahierenden Lentikels ermittelt werden (S4), die dann also dasjenige Stück formen, das vor, während oder nach der Drehung entfernt, also extrahiert, werden soll. Anders definiert und mit anderen Worten kann hierzu alternativ ein Lentikelbereich des zu drehenden Lentikels 12 ermittelt werden, wobei der ermittelte Lentikelbereich dann vor, während oder nach der Drehung auf dem Fachmann aus dem Stand der Technik bekannter Weise zur Extraktion entfernt werden kann.

Das Extrahieren eines Lentikelbereichs kann zum Beispiel vorgesehen werden, um eine Hornhautverkrümmung zu reduzieren oder sogar zu beheben, und dabei die Hornhaut an der Stelle, an der der Lentikelbereich extrahiert wird, abzuflachen. Zum Betreiben der Behandlungsvorrichtung 10, insbesondere der Behandlungsvorrichtung 10 der Fig. 1, kann die Steuereinrichtung 20 dann die bereitgestellten Steuerdaten an den augenchirurgischen Laser 18 der Behandlungsvorrichtung 10 übertragen (S8).

Die Fig. 4 zeigt ein Beispiel, in dem die Kornea 17 zum Beispiel eine eiförmige Verkrümmung aufweisen kann. Bei zum Beispiel einer Drehung von 90 Grad eines Lentikels 12 kann beispielhaft eine Hälfte der Abflachung erzielt werden, die Verkrümmung also ausgeglichen werden. Die Soll-Geometrie mit einer flacheren Krümmung ist in der Fig. 4 als Krümmung 48 schematisiert. Die Verkrümmung, die gemäß der Ist-Geometrie die Hornhautpathologie vor dem Eingriff erzeugt, ist durch die Linien 50 gezeigt.

Am Beispiel der Fig. 4 ist das optionale Markieren eines Lentikelbereichs und/oder eines Bereichs der Kornea 17 außerhalb des Lentikels 12 zum exakten Positionieren des Lentikels 12 veranschaulicht. Eine solche Markierung oder solche Markierungen können optional auch im Beispiel der Fig. 3 durchgeführt werden. Aufgrund der besseren Übersichtlichkeit ist dies jedoch im Folgenden nur zur Fig. 4 beschrieben.

In der Fig. 4 ist der Lentikel 12 hierzu stark vereinfacht dargestellt. Durch beispielsweise eine Drehung D von 90 Grad soll zum Beispiel ein Lentikelbereich 52 nach der Drehung an einer Soll-Endposition stehen, die zum Beispiel dadurch festgestellt werden kann (S9), indem exakte Koordinaten der Soll-Endposition an zum Beispiel dem oben erwähnten beispielhaften dreidimensionalen Modell des Korrekturbereichs 32 ermittelt werden können. Die Steuereinrichtung 20 kann die Soll-Endposition und/oder den Lentikelbereich 52, insbesondere Koordinaten einer Referenzstelle im Lentikelbereich 52, ermitteln. Die bereitgestellten Steuerdaten können dann vorgeben, dass der Augenlaser 18 an den Koordinaten der Stelle im Lentikelbereich 52 und/oder an der Soll-Endposition jeweils eine Markierung einbringen, zum Beispiel durch Einlasern eines Striches im Lentikel 12, und/oder durch Einlasern eines Striches in zum Beispiel dem Epithel des Restkörpers der Kornea 17. Vorzugsweise kann zum Beispiel die Markierung, die im Epithel der Kornea 17 angebracht wird, also im Restkörper der Kornea 17, ein Kreis sein, und die Markierung 54 im Lentikelbereich 52 kann dann zum Beispiel ein gelasertes Kreuz sein. Die Drehung D kann dann erkennbar um den vorgegebenen Drehwinkel erfolgt sein, wenn die Markierung 54 des Lentikelbereichs 52 mit der Markierung 54 im Epithel überlappt. Optional können beide Markierungen 54 gelaserte Fadenkreuze sein.

Mit anderen Worten kann eine Markierung 54 physikalisch auf dem Lentikel 12 angebracht werden. Zusätzlich oder alternativ kann eine Markierung 54 an der End-Sollposition angebracht werden. Wird zum Beispiel nur eine Markierung 54 auf dem Lentikel 12 angebracht, so kann zum Beispiel mittels einer Kamera der Korrekturbereich 32 während der Drehung D überwacht werden, und die End-Sollposition in der Kornea 17 kann zum Beispiel als graphische Darstellung auf einem Bildschirm eingezeichnet sein.

Insgesamt veranschaulichen die Beispiele, wie durch die Erfindung eine Lentikel-Rotation ermöglicht und durchgeführt werden kann.

Gemäß einem weiteren Ausführungsbeispiel kann ein Verfahren für eine Korrektur, insbesondere eine besondere Korrektur, vorzugsweise für eine Korrektur oder Korrekturen, die nicht rotationssymmetrisch sind, bereitgestellt werden, wobei der Lentikel 12 in einer Art und Weise geschnitten wird, dass er nicht extrahiert werden muss, aber um ein Zentrum, also um einen Drehbereich 46 oder einen Drehpunkt rotiert wird. Eine Behandlungsvorrichtung 10 kann hierzu:
1) für eine gegebene Vorschrift einer Korrektur die adäquate, also passende Lentikelform finden, die benötigt wird zum Korrigieren, zum Beispiel zum Korrigieren der Vorschrift, durch Rotation und nicht durch Extraktion; und/oder
2) weiterhin kann das System die Weite der Rotation bestimmen, also den Drehwinkel vorgeben, die/der benötigt wird, um den Lentikel 12 so zu rotieren, dass die unrotierte Vorschrift korrigiert wird; und/oder
3) weiterhin kann das System dazu eingerichtet sein, einen solchen Schnitt 34 oder solche Schnitte 34 durchzuführen, um diesen Lentikel 12 zu generieren; und/oder
4) weiterhin kann das System dazu eingerichtet sein, einen oder mehrere Markierungen 54 bereitzustellen, um die Rotation, insbesondere durch einen Chirurgen, zu erleichtern:
   a) eine Markierung 54 an der kornealen Oberfläche/in dem Epithel als Zielmarkierung; diese kann zum Beispiel ein Schnitt sein, um die Rotation des Lentikels 12 zu erleichtern; und/oder
   b) eine weitere Markierung 54, vorzugsweise an der Peripherie der Lentikeloberfläche als Rotationsmarkierung; und/oder
   c) zum Beispiel kann ein Chirurg die Markierung 54 auf dem Lentikel 12 mit der Markierung 54 auf dem Epithel überlagern; dadurch ergeben sich eine genaue Rotation und damit die Korrektur.

Gemäß einem weiteren, besonders bevorzugten Ausführungsbeispiel kann das System, insbesondere die Behandlungsvorrichtung 10, einen zentralen Bereich, also zum Beispiel den Drehbereich 46, "ungeschnitten" lassen, sodass dieser als Drehpunkt dient. Vorzugsweise kann das System, insbesondere die Behandlungsvorrichtung 10, Möglichkeiten zum Bestimmen, ob eine Rotation des Lentikels 12 ausreicht, umfassen, oder ob ein Kombinationsansatz gefolgt werden soll, in dem zwei Lentikel 12 oder zwei Lentikelanteile verbunden und/oder ermittelt werden (vorzugsweise beide kleiner als ein konventioneller Lentikel 12), wobei einer rotiert werden kann, und der andere extrahiert.

Im Folgenden werden bevorzugte Ausführungsbeispiele mit genauen Abmessungen beziehungsweise beispielhaften Fehlsichtigkeitsdaten beschrieben.

Im ersten Ausführungsbeispiel kann die Stärke der Refraktion beispielhaft + 3,5 - 7,0 Dx 0 sein (medizinische Notation der Stärke der Refraktion: SCA, also Sphäre, Zylinder, Achse).

Ein Zylinder (torische Linse, "C") beschreibt eine "relative" Krümmung, während eine Sphäre (sphärische Linse, "S") eine absolute Krümmung beschreibt. Die absoluten Krümmungen sind also S in der A Achslage; S+C in der Achslage senkrecht zur A Achslage; und dazwischen ist die Stärke S+C/2*(1-cos(2*(achse-A))).

Im Beispiel kann also die Stärke der Refraktion sein: eine Sphäre von +3,5D, ein Zylinder beziehungsweise zylindrische Form von -7,0D, und eine Rotationsachse ("A") des Zylinders von 0deg, also 0 Grad.

Im Beispiel kann die Stärke der Refraktion auch als - 3,5 + 7x90 beschrieben werden, sie wirkt identisch. Mit anderen Worten kann bei 0 Grad die Stärke +3,5D sein (bei 90 Grad +3,5-7=-3,5D).

Im Beispiel kann zur Kompensation etwas ermittelt werden, zum Beispiel ein zentraler Lentikel 12, der bei 0 Grad eine Stärke von +3,5D hat, und bei 90grad Null Stärke. Das kann insbesondere 0+3.5x90 sein, also S=0D, C=+3,5D, A=90deg (bei 90 Grad 0D Stärke, und bei 0 Grad 0+3,5=+3,5D Stärke). Wenn dies um 90 Grad gedreht wird, dann wirkt es als 0+3.5x0. Wenn dazu das "inseriert" statt entfernt wird, dann wirkt es als 0-3,5x0 (S=0D; C=-3,5D; A=0deg (bei 0grad 0D Stärke, und bei 90grad 0-3,5=-3,5D Stärke)).

Durch die Kombination wirkt das bei 0 Grad als +3,5D+0D=+3,5D Stärke, bei 90 Grad als 0-3,5=-3,5D Stärke, also wie gewünscht +3,5-7x0.

Alternativ bei einer Stärke von +3,5-7=-3,5D bei 90 Grad und bei 0 Grad null Stärke, wäre das 0-3,5x0 (S=0D; C=-3,5D; A=0deg, bei 0 Grad 0D Stärke und bei 90grad 0-3.5=-3.5D Stärke. Wenn dies um 90 Grad gedreht wird, dann wirkt es als 0-3,5x90. Wenn dazu "inseriert" wird, anstatt zu entfernen, dann wirkt es als 0+3,5x90 (S=0D; C=+3,5D; A=90deg, bei 90 Grad 0D Stärke und bei 0 Grad 0+3,5=+3,5D Stärke).

Durch die Kombination wirkt dies also bei 0 Grad als +3,5D+0D=+3,5D Stärke, und bei 90 Grad 0-3,5=-3,5D Stärke, also wie gewünscht +3,5-7x0.

Dieses Beispiel für +3,5-7,0Dx0 kann noch einmal wie folgt zusammengefasst werden:
1) Das System kann entweder eine um 90deg zu rotierende 0+3,5Dx90 durchführen,
2) oder eine 0-3,5Dx0, um 90deg (also 90Grad) zu rotieren,
3) die +3,5-7,0Dx0 in 7mm (Millimetern) nimmt 65µm (Mikrometer) zentral, 135µm maximale Tiefe, und 3700nl (Nanoliter) Volumen
   1) The 0+3,5Dx90 in 7mm nimmt 0µm zentral (-100%), 65µm, maximale Tiefe (-50%), und 1400nl Volumen (-60%)
   2) The 0-3,5Dx0 in 7mm nimmt 65µm zentral (-0%), 65µm maximale Tiefe (-50%), und 2300nl Volumen (-40%)
   3) Kein Gewebe wird extrahiert

In einem weiteren Beispiel mit einer Refraktionsstärke von + 2,0 - 6,0 Dx0 kann analog:
1) das System entweder eine um 90deg zu rotierende 0+3,0Dx90 durchführen,
2) oder eine 0-3,0Dx0 um 90deg (also 90Grad) rotieren,
3) plus eine -1,0D zu extrahierende Sphäre,
4) die +3,0-6,0Dx0 in 7mm nimmt 55µm zentral, 115µm maximale Tiefe, und 3100nl Volumen
   1) Die 0+3,0Dx90 in 7mm nimmt 0µm zentral, 55µm maximale Tiefe, und 1200nl Volumen
   2) die 0-3,0Dx0 in 7mm nimmt 55µm zentral, 55µm maximale Tiefe, und 1900nl Volumen
   3) die -1,0D in 7mm nimmt 15µm zentral (-70%), 15µm maximale Tiefe (-85%), und 350nl Volumen (-85%). Nur dieses kann dann extrahiert werden.

In einem weiteren Beispiel zur Behandlung eines Keratokonus (vergleiche beispielsweise Fig. 3) kann das System, insbesondere die Behandlungsvorrichtung 10, vorzugsweise derart gesteuert werden, dass sie eine Behandlung für zum Beispiel die Hälfte des Komas durchführt, das um beispielsweise 180 Grad zu rotieren ist. Vorzugsweise kann das System, insbesondere die Behandlungsvorrichtung 10, Markierungen 54 am Lentikel 12 und/oder an zum Beispiel den Epithelen anbringen, die zum Beispiel 180 Grad gegenüberliegen.

Gemäß einem weiteren Ausführungsbeispiel können Steuerdaten für ein generisches Behandeln eines Keratokonus bereitgestellt werden. Das System, insbesondere die Behandlungsvorrichtung 10, kann dabei derart gesteuert werden, dass sie eine Behandlung für die Hälfte des Komas durchführt, das zum Beispiel um 180 Grad zu rotieren ist. Es können Markierungen 54 zum Beispiel 180 Grad voneinander entfernt oder gegenüberliegend angebracht werden, vorzugsweise auf dem Lentikel 12 und im Epithel. Zusätzlich kann eine myopische Sphäre extrahiert werden, vorzugsweise eine kleine myopische Sphäre.

Gemäß einem weiteren Ausführungsbeispiel kann durch die Behandlungsvorrichtung 10 die Behandlung eines kleeblattförmigen Abriebs der Kornea 17 durch das Lid behandelt werden ("Trefoil"). Das System, insbesondere die Behandlungsvorrichtung 10, können dabei derart gesteuert werden, dass es eine Behandlung entweder der Hälfte des "Kleeblatts" (also zu korrigierende Stellen, die in einem Winkel von zirka 120 Grad zueinander stehen) durchführt, unter Rotation um 60 Grad. Zusätzlich können Markierungen 54 in einem Winkel von 60 Grad zueinander im Lentikel 12 und Epithelen angebracht werden.

Gemäß einem weiteren Ausführungsbeispiel kann eine generische Behandlung einer solchen Kleeblatt-Struktur durchgeführt werden. Das System, insbesondere die Behandlungsvorrichtung 10, kann hierbei zum Beispiel die Hälfte des "Kleeblatts", das um vorzugsweise 60 Grad gedreht werden soll, durchführen. Markierungen 54, die in einem Winkel von 60 Grad zueinander stehen, können im Lentikel 12 und Epithelium angebracht werden. Vorzugsweise kann zusätzlich eine Sphäre extrahiert werden, also ein Lentikelbereich, insbesondere ein kleiner Lentikelbereich.

## Patentansprüche

1. Verfahren zum Bereitstellen von Steuerdaten für einen augenchirurgischen Laser, wobei das Verfahren wenigstens die folgenden, durch mindestens eine Steuereinrichtung (20) durchgeführten Schritte aufweist:
- Feststellen von Fehlsichtigkeitsdaten, die einen Grad einer Fehlsichtigkeit eines menschlichen oder tierischen Auges (40) beschreiben (S1),
- Feststellen einer Ist-Geometrie eines Korrekturbereichs (32) einer Kornea (17) des Auges (40), die dreidimensionale Abmessungen des Korrekturbereichs (32) der Kornea (17) beschreibt (S2),
- anhand der festgestellten Fehlsichtigkeitsdaten und anhand der festgestellten Ist-Geometrie: Ermitteln einer Soll-Geometrie des Korrekturbereichs (32), die einen Reduktionsanteil (44) des Korrekturbereichs (32) mit einer gegenüber der Ist-Geometrie verminderten Kornea-Dicke beschreibt, und/oder einen Verdickungsanteil (42) mit einer gegenüber der Ist-Geometrie erhöhten Kornea-Dicke; wobei die ermittelte Soll-Geometrie ein vorgegebenes Fehlsichtigkeitsreduktions-Kriterium erfüllt, das eine Eignung zur Reduktion des Grads der Fehlsichtigkeit vorgibt (S3),
- anhand der festgestellten Ist-Geometrie: Ermitteln von Grenzflächen (14, 16) eines Lentikels (12) innerhalb des Korrekturbereichs (32), der sich zumindest teilweise über den Korrekturbereich (32) erstreckt, wobei ein Anteil des Lentikels (12) derart ausgestaltet ist, dass er bei einer Drehung (D) des Lentikels (12) in den Verdickungsanteil (42) um eine vorgegebene, durch die Kornea (17) verlaufende Drehachse (31) und um einen vorgegebenen Drehwinkel den Korrekturbereich (32) von der festgestellten Ist-Geometrie in die ermittelte Soll-Geometrie überführt (S4), und
- Bereitstellen von Steuerdaten, die die ermittelten Grenzflächen (14, 16) zum Formen des Lentikels (12) beschreiben (S6), **dadurch gekennzeichnet, dass** die ermittelten Grenzflächen (14, 16) den Lentikel (12) nur außerhalb eines Drehbereichs (46) des Lentikels (12), durch den die vorgegebene Drehachse verläuft, aus dem Korrekturbereich (32) der Kornea (17) abtrennen, sodass der Lentikel (12) im Drehbereich (46) mit dem an der Lederhaut verbundenen Körper der Kornea (17) verbunden bleibt.

2. Verfahren nach Anspruch 1, **dadurch gekennzeichnet, dass** die bereitgestellten Steuerdaten die vorgegebene Drehachse (31) und eine Rotation des Lentikels (12) um den vorgegebenen Drehwinkel beschreiben.

3. Verfahren nach einem der vorhergehenden Ansprüche, **dadurch gekennzeichnet, dass** die Steuereinrichtung (20) durchführt:
- Festlegen von mindestens einem Inzisionsverlauf, der sich jeweils von einer vorgegebenen Inzisionsstelle einer Außenseite der Kornea (17) zu einer der festgelegten Grenzflächen (14, 16) hin erstreckt (S7),
wobei die bereitgestellten Steuerdaten den mindestens einen ermittelten Inzisionsverlauf beschreiben.

4. Verfahren nach einem der vorhergehenden Ansprüche, **dadurch gekennzeichnet, dass** die Steuereinrichtung (20) durchführt:
- Vorgeben des Drehwinkels und/oder der Drehachse (31) in Abhängigkeit von:
a) einem Ergebnis eines Vergleichs der festgestellten Ist-Geometrie mit der ermittelten Soll-Geometrie, und/oder b) den festgestellten Fehlsichtigkeitsdaten (S5).

5. Verfahren nach einem der vorhergehenden Ansprüche, **dadurch gekennzeichnet, dass** die Steuereinrichtung (20) eine Soll-Endposition eines vorgegebenen Lentikelbereichs (52) des gedrehten Lentikels (12) feststellt (S9), und wobei:
- die bereitgestellten Steuerdaten ein Markieren der festgestellten Soll-Endposition außerhalb des Lentikels (12) beschreiben; und/oder wobei
- die bereitgestellten Steuerdaten ein Markieren des vorgegebenen Lentikelbereichs (52) beschreiben.

6. Verfahren nach Anspruch 5, **dadurch gekennzeichnet, dass** die bereitgestellten Steuerdaten ein Markieren des Lentikelbereichs (52) an dessen Koordinaten durch einen Augenlaser beschreibt.

7. Verfahren nach einem der vorhergehenden Ansprüche, **dadurch gekennzeichnet, dass** die Grenzflächen (14, 16) den Lentikel (12) von der Kornea (17) vollständig abtrennen.

8. Verfahren nach einem der vorhergehenden Ansprüche, **dadurch gekennzeichnet, dass** die Steuereinrichtung (20) zur Ausführung der folgenden Verfahrensschritte ausgebildet ist:
- Ermitteln von Grenzflächen (14, 16) eines weiteren, zu extrahierenden Lentikels (12) oder eines zu extrahierenden Lentikelbereichs (52) des zu drehenden Lentikels (12) anhand eines Ergebnisses eines Vergleichs der ermittelten Soll-Geometrie und der festgestellten Ist-Geometrie, der sich vorzugsweise nur im Reduktionsanteil (44) befindet; wobei die bereitgestellten Steuerdaten die Grenzflächen (14, 16) des weiteren Lentikels (12) oder des zu extrahierenden Lentikelbereichs (52) beschreiben (S4).

9. Verfahren nach einem der vorhergehenden Ansprüche, **dadurch**
**gekennzeichnet, dass** die Steuereinrichtung (20) dazu eingerichtet ist den Laser (18) derart zu steuern,
dass er Laserpulse in einem Wellenlängenbereich zwischen 300 nm und 1400 nm, vorzugsweise zwischen 700 nm und 1200 nm, bei einer jeweiligen Pulsdauer zwischen 1 fs und 1 ns, vorzugsweise zwischen 10 fs und 10 ps, und einer Wiederholungsfrequenz größer 10 KHz, vorzugsweise zwischen 100 KHz und 100 MHz, abgibt.

10. Steuereinrichtung (20), die dazu eingerichtet ist, ein Verfahren nach einem der vorhergehenden Ansprüche durchzuführen.

11. Behandlungsvorrichtung (10) mit mindestens einem augenchirurgischen Laser (18) für die Abtrennung eines Hornhautvolumens mit vordefinierten Grenzflächen (14, 16) eines menschlichen oder tierischen Auges (40) mittels Photodisruption und mindestens einer Steuereinrichtung (20) nach Anspruch 10.

12. Behandlungsvorrichtung (10) nach Anspruch 11, **dadurch gekennzeichnet, dass** der Laser (18) geeignet ist, Laserpulse in einem Wellenlängenbereich zwischen 300 nm und 1400 nm, vorzugsweise zwischen 700 nm und 1200 nm, bei einer jeweiligen Pulsdauer zwischen 1 fs und 1 ns, vorzugsweise zwischen 10 fs und 10 ps, und einer Wiederholungsfrequenz größer 10 KHz, vorzugsweise zwischen 100 KHz und 100 MHz, abzugeben.

13. Behandlungsvorrichtung (10) nach Anspruch 11 oder 12, **dadurch gekennzeichnet, dass** die Steuereinrichtung (20):
- mindestens eine Speichereinrichtung zur zumindest temporären Speicherung von mindestens einem Steuerdatensatz aufweist, wobei der oder die Steuerdatensätze Steuerdaten zur Positionierung und/oder zur Fokussierung einzelner Laserpulse in der Hornhaut umfassen; und
- mindestens eine Strahleinrichtung (22) zur Strahlführung und/oder Strahlformung und/oder Strahlablenkung und/oder Strahlfokussierung eines Laserstrahls (24) des Lasers (18) umfasst.

14. Computerprogramm, umfassend Befehle, die bewirken, dass die Behandlungsvorrichtung (10) gemäß einem der Ansprüche 11 bis 13 ein Verfahren nach einem der Ansprüche 1 bis 9 ausführt.

15. Computerlesbares Medium, auf welchem das Computerprogramm nach Anspruch 14 gespeichert ist.

## Claims

1. A method for providing control data for an eye surgical laser, wherein the method comprises at least the following steps performed by at least one control device (20):
- determining visual disorder data, which describes a degree of a visual disorder of a human or animal eye (40) (S1),
- determining an actual geometry of a correction area (32) of a cornea (17) of the eye (40), which describes three-dimensional dimensions of the correction area (32) of the cornea (17) (S2),
- based on the determined visual disorder data and based on the determined actual geometry: ascertaining a target geometry of the correction area (32), which describes a reduction portion (44) of the correction area (32) with a corneal thickness reduced with respect to the actual geometry, and/or a thickening portion (42) with a corneal thickness increased with respect to the actual geometry; wherein the ascertained target geometry satisfies a preset visual disorder reduction criterion, which presets a suitability for reducing the degree of the visual disorder (S3),
- based on the determined actual geometry: ascertaining interfaces (14, 16) of a lenticule (12) within the correction area (32), which at least partially extends across the correction area (32), wherein a portion of the lenticule (12) is configured such that it transfers the correction area (32) from the determined actual geometry into the ascertained target geometry upon a rotation (D) of the lenticule (12) into the thickening portion (42) around a preset rotational axis (31) extending through the cornea (17) and by a preset rotational angle (S4), and
- providing control data, which describes the ascertained interfaces (14, 16) for shaping the lenticule (12) (S6), **characterized in that** the ascertained interfaces (14, 16) separate the lenticule (12) out of the correction area (32) of the cornea (17) only outside of a rotational area (46) of the lenticule (12), through which the preset rotational axis extends, such that the lenticule (12) remains connected to the body of the cornea (17) connected to the sclera in the rotational area (46).

2. The method according to claim 1, **characterized in that** the provided control data describes the preset rotational axis (31) and a rotation of the lenticule (12) by the preset rotational angle.

3. The method according to any one of the preceding claims, **characterized in that** the control device (20) performs:
- setting at least one incision course, which each extends from a preset incision location of an outer side of the cornea (17) towards one of the determined interfaces (14, 16) (S7),
wherein the provided control data describes the at least one ascertained incision course.

4. The method according to any one of the preceding claims, **characterized in that** the control device (20) performs:
- presetting the rotational angle and/or the rotational axis (31) depending on:
a) a result of a comparison of the determined actual geometry to the ascertained target geometry, and/or b) the determined visual disorder data (S5).

5. The method according to any one of the preceding claims, **characterized in that** the control device (20) determines a target final position of a preset lenticule area (52) of the rotated lenticule (12) (S9), and wherein:
- the provided control data describes marking of the determined target final position outside of the lenticule (12); and/or wherein
- the provided control data describes marking of the preset lenticule area (52).

6. The method according to claim 5, **characterized in that** the provided control data describes marking of the lenticule area (52) at the coordinates thereof by an eye laser.

7. The method according to any one of the preceding claims, **characterized in that** the interfaces (14, 16) completely separate the lenticule (12) from the cornea (17).

8. The method according to any one of the preceding claims, **characterized in that** the control device (20) is formed for executing the following method steps:
- ascertaining interfaces (14, 16) of a further lenticule (12) to be extracted or of a lenticule area (52) to be extracted of the lenticule to be rotated (12) based on a result of a comparison of the ascertained target geometry and the determined actual geometry, which is preferably situated only in the reduction portion (44); wherein the provided control data describes the interfaces (14, 16) of the further lenticule (12) or of the lenticule area (52) to be extracted (S4).

9. The method according to any one of the preceding claims, **characterized in that** the control device (20) is configured to control the laser (18) such that it emits laser pulses in a wavelength range between 300 nm and 1400 nm, preferably between 700 nm and 1200 nm, at a respective pulse duration between 1 fs and 1 ns, preferably between 10 fs and 10 ps, and a repetition frequency of greater than 10 kHz, preferably between 100 kHz and 100 MHz.

10. A control device (20), which is configured to perform a method according to any one of the preceding claims.

11. A treatment apparatus (10) with at least one eye surgical laser (18) for the separation of a corneal volume with predefined interfaces (14, 16) of a human or animal eye (40) by means of photodisruption, and at least one control device (20) according to claim 10.

12. The treatment apparatus (10) according to claim 11, **characterized in that** the laser (18) is suitable to emit laser pulses in a wavelength range between 300 nm and 1400 nm, preferably between 700 nm and 1200 nm, at a respective pulse duration between 1 fs and 1 ns, preferably between 10 fs and 10 ps, and a repetition frequency of greater than 10 kHz, preferably between 100 kHz and 100 MHz.

13. The treatment apparatus (10) according to claim 11 or 12, **characterized in that** the control device (20):
- comprises at least one storage device for at least temporary storage of at least one control dataset, wherein the control dataset or datasets include(s) control data for positioning and/or for focusing individual laser pulses in the cornea; and
- includes at least one beam device (22) for beam guidance and/or beam shaping and/or beam deflection and/or beam focusing of a laser beam (24) of the laser (18).

14. A computer program comprising commands, which cause the treatment apparatus (10) according to any one of claims 11 to 13 to execute a method according to any one of claims 1 to 9.

15. A computer-readable medium, on which the computer program according to claim 14 is stored.

## Revendications

1. Procédé pour fournir des données de commande pour un laser chirurgical ophtalmique, le procédé comportant au moins les étapes suivantes réalisées par au moins un dispositif de commande (20) :
- établir des données concernant un trouble visuel qui décrivent (S1) un degré de trouble visuel d'un oeil humain ou animal (40),
- établir une géométrie réelle d'une région de correction (32) d'une cornée (17) de l'œil (40), qui décrit (S2) les dimensions tridimensionnelles de la région de correction (32) de la cornée (17),
- au moyen des données établies concernant un trouble visuel et au moyen de la géométrie réelle établie : déterminer une géométrie cible de la région de correction (32), qui décrit une partie de réduction (44) de la région de correction (32) ayant une épaisseur de cornée réduite par rapport à la géométrie réelle, et/ou une partie d'épaississement (42) ayant une épaisseur de cornée augmentée par rapport à la géométrie réelle ; la géométrie cible déterminée satisfaisant à un critère prédéterminé de réduction de trouble visuel qui spécifie une aptitude à réduire le degré de trouble visuel (S3),
- au moyen de la géométrie réelle établie : déterminer des interfaces (14, 16) d'un lenticule (12) à l'intérieur de la région de correction (32), qui s'étend au moins partiellement à travers la région de correction (32), une partie du lenticule (12) étant conçue de telle sorte que lors d'une rotation (D) du lenticule (12) dans la partie d'épaississement (42) autour d'un axe de rotation (31) prédéterminé s'étendant à travers la cornée (17) et selon un angle de rotation prédéterminé, la région de correction (42) est transférée (S4) de la géométrie réelle établie à la géométrie cible déterminée, et
- fournir des données de commande qui décrivent (S6) les interfaces (14, 16) déterminées pour la mise en forme du lenticule (12), **caractérisé en ce que** les interfaces (14, 16) déterminées séparent le lenticule (12) uniquement à l'extérieur d'une zone de rotation (46) du lenticule (12) à travers laquelle l'axe de rotation prédéterminé s'étend, de la région de correction (32) de la cornée (17), de sorte que le lenticule (12) reste relié au corps de la cornée (17) relié à la sclérotique dans la zone de rotation (46).

2. Procédé selon la revendication 1, **caractérisé en ce que** les données de commande fournies décrivent l'axe de rotation (31) prédéterminé et une rotation du lenticule (12) suivant de l'angle de rotation prédéterminé.

3. Procédé selon l'une des revendications précédentes, **caractérisé en ce que** le dispositif de commande (20) effectue :
- la détermination d'au moins un trajet d'incision qui s'étend (S7) respectivement à partir d'un site d'incision prédéterminé d'un côté extérieur de la cornée (17) jusqu'à l'une des interfaces (14, 16) déterminées,
les données de commande fournies décrivant au moins un trajet d'incision déterminé.

4. Procédé selon l'une des revendications précédentes, **caractérisé en ce que** le dispositif de commande (20) réalise :
- la spécification de l'angle de rotation et/ou de l'axe de rotation (31) en fonction : a) d'un résultat d'une comparaison de la géométrie réelle établie avec la géométrie cible déterminée, et/ou b) des données établies concernant un trouble visuel (S5).

5. Procédé selon l'une des revendications précédentes, **caractérisé en ce que** le dispositif de commande (20) établit (S9) une position cible finale d'une région lenticulaire prédéterminée (52) du lenticule tourné (12), et :
- les données de commande fournies décrivant un marquage de la position cible finale établie à l'extérieur du lenticule (12) ; et/ou
- les données de commande fournies décrivant un marquage de la région lenticulaire (52) spécifiée.

6. Procédé selon la revendication 5, **caractérisé en ce que** les données de commande fournies décrivent, au moyen d'un laser ophtalmique, un marquage de la région lenticulaire (52) par ses coordonnées.

7. Procédé selon l'une des revendications précédentes, **caractérisé en ce que** les interfaces (14, 16) séparent entièrement le lenticule (12) de la cornée (17).

8. Procédé selon l'une des revendications précédentes, **caractérisé en ce que** le dispositif de commande (20) est conçu pour réaliser les étapes de procédé suivantes :
- déterminer des interfaces (14, 16) d'un autre lenticule (12) à extraire ou d'une région lenticulaire (52) à extraire du lenticule (12) à faire tourner au moyen d'un résultat d'une comparaison de la géométrie cible établie et de la géométrie réelle déterminée qui se situe de préférence uniquement dans la partie de réduction (44) ; les données de commande fournies décrivant (S4) les interfaces (14, 16) de l'autre lenticule (12) ou de la région lenticulaire (52) à extraire.

9. Procédé selon l'une des revendications précédentes, **caractérisé en ce que** le dispositif de commande (20) est conçu pour commander le laser (18), de telle sorte qu'il émet des impulsions laser dans une gamme de longueurs d'onde comprise entre 300 nm et 1 400 nm, de préférence entre 700 nm et 1 200 nm, à une durée d'impulsion respective comprise entre 1 fs et 1 ns, de préférence entre 10 fs et 10 ps, et une fréquence de répétition supérieure à 10 kHz, de préférence entre 100 kHz et 100 MHz.

10. Dispositif de commande (20) configuré pour mettre en oeuvre un procédé selon l'une des revendications précédentes.

11. Dispositif de traitement (10) avec au moins un laser chirurgical ophtalmique (18) pour la séparation d'un volume de cornée avec des interfaces (14, 16) prédéfinies d'un oeil humain ou animal (40) au moyen d'une photodisruption, et au moins un dispositif de commande (20) selon la revendication 10.

12. Dispositif de traitement (10) selon la revendication 11, **caractérisé en ce que** le laser (18) est adapté pour émettre des impulsions laser dans une gamme de longueurs d'onde comprise entre 300 nm et 1 400 nm, de préférence entre 700 nm et 1 200 nm, à une durée d'impulsion respective comprise entre 1 fs et 1 ns, de préférence entre 10 fs et 10 ps, et une fréquence de répétition supérieure à 10 kHz, de préférence entre 100 kHz et 100 MHz.

13. Dispositif de traitement (10) selon la revendication 11 ou 12, **caractérisé en ce que** le dispositif de commande (20) :
- comporte au moins un dispositif de stockage pour le stockage au moins temporaire d'au moins un ensemble de données de commande, l'ensemble ou les ensembles de données de commande comprenant des données de commande pour le positionnement et/ou la focalisation d'impulsions laser individuelles dans la cornée ; et
- comprend au moins un dispositif à faisceau (22) pour le guidage de faisceau et/ou la conformation de faisceau et/ou la déviation de faisceau et/ou la focalisation de faisceau d'un faisceau laser (24) du laser (18).

14. Programme informatique comprenant des instructions qui amènent le dispositif de traitement (10) selon l'une des revendications 11 à 13 à mettre en oeuvre un procédé selon l'une des revendications 1 à 9.

15. Support lisible par ordinateur sur lequel est mémorisé le programme informatique selon la revendication 14.
